# EUROPEAN PATENT APPLICATION

(11) **EP 4 807 682 A1**
(43) Date of publication of application: **16.09.2026**
(21) Application number: 26163955.3
(22) Date of filing: 11.03.2026
(51) Int. Cl.: G06T 15/08, G06T 15/50, G16H 50/00

(54) **ASSISTED PLACEMENT OF VIRTUAL LIGHT SOURCES IN MEDICAL IMAGE DATA**

(30) Priority: 13.03.2025 US 202519078745
(71) Applicant: Siemens Medical Solutions USA, Inc., Malvern, PA 19355-1406 (US)
(72) Inventor: SMITH-CASEM, Mervin Mencias, Issaquah, WA, 98029-7002 (US); VARCHOLA, Andrej, 040 11 Kosice (SK); KARIMOV, Alexey, 1210 Wien (AT)
(74) Representative: HKW Intellectual Property PartG mbB

(57) **Abstract**

A framework for assisted placement of virtual light sources in medical image data. The framework receives (204) medical image data of at least one structure of interest and calculates (206) at least one geometrical feature of the at least one structure of interest based on the medical image data. An optimal position of at least one virtual light source is determined (208) near or within the structure of interest using the geometrical feature. Volume rendering (210) of the medical image data is performed using the at least one virtual light source at the optimal position to generate a projection image.

## Description

### TECHNICAL FIELD

The present disclosure generally relates to medical imaging, and more particularly to a framework for assisted placement of virtual light sources in medical image data.

### BACKGROUND

Diagnostic medical modalities, such as computed tomography (CT), magnetic resonance (MR) and ultrasound, may be used to acquire detailed volumetric (i.e., three-dimensional or 3D) medical images. The medical images depict anatomical structures, such as internal organs and tumors. Virtual lights in volumetric medical imaging may be used to improve depth perception in the volume rendered images by adding shadows and highlights to anatomical structures in the volumetric image data. A virtual point light is located at a point in the volumetric image data space and emits light in all directions equally, whereas a virtual directional light is located infinitely far away and emits light in one direction only.

Virtual point lights are especially useful when they are placed inside fluid-filled structures that may be difficult to illuminate with a virtual directional light. Virtual point lights may also be used to determine if there are any holes in anatomical structures (e.g., cardiac valves) by placing a virtual point light on side of the structure and looking for leakage of the virtual light on the other side, similar to using color Doppler.

Traditionally, virtual lights are manually placed, or placed in preset locations, near or within volumetric data sets of anatomical structures. Performing such manual placement on medical ultrasound scanners or workstations can be cumbersome and challenging, because the display system is typically two-dimensional and the user controls usually only provide control over one or two dimensions. For example, the user manually places the virtual point lights manually using mouse-based or trackball-based user controls, possibly in conjunction with other use controls such as knobs. This manual placement can be even more challenging if the volumetric data is updating in real-time during live imaging or cine playback. Placing multiple point lights within the volumetric data set will further increase the complexity and time required for completing the workflow.

### SUMMARY

Described herein is a framework for assisted placement of virtual light sources in medical image data. The framework receives medical image data of at least one structure of interest and calculates at least one geometrical feature of the at least one structure of interest based on the medical image data. An optimal position of at least one virtual light source is determined near or within the structure of interest using the geometrical feature. Volume rendering of the medical image data is performed using the at least one virtual light source at the optimal position to generate a projection image.

### BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings.
FIG. 1 is a block diagram illustrating an exemplary imaging system;
FIG. 2 shows an exemplary method of medical image visualization;
FIGS. 3a and 3b show an exemplary four-dimensional (4D) ultrasound cardiac image;
FIG. 4 shows an exemplary method of automatically determining the centerline of the structure of interest;
FIG. 5a shows an exemplary projection image of a heart; and
FIG. 5b shows another exemplary projection image of a heart.

### DETAILED DESCRIPTION

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order-dependent in their performance.

Unless stated otherwise as apparent from the following discussion, it will be appreciated that terms such as "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

One aspect of the present framework automatically places virtual light sources within medical image data. One or more virtual light sources may be automatically placed as close as possible to one or more desired locations near or within the anatomical structures of interest to minimize the amount of manual position adjustments required from the user. The user may select one or more anatomical structures of interest to be illuminated by the virtual light(s). Virtual light source positions may be calculated to be near or inside anatomical structures of interest. Such positions may be automatically updated periodically in real-time. The virtual light sources may then be automatically placed at the computed positions within the medical image data volume. The user may adjust the position(s) of the virtual light source(s). Assisted placement of virtual light sources enabled by the present framework simplifies and accelerates the user's workflow by eliminating or minimizing manual virtual light source positioning. These and other exemplary advantages and features will be described in more details in the following description.

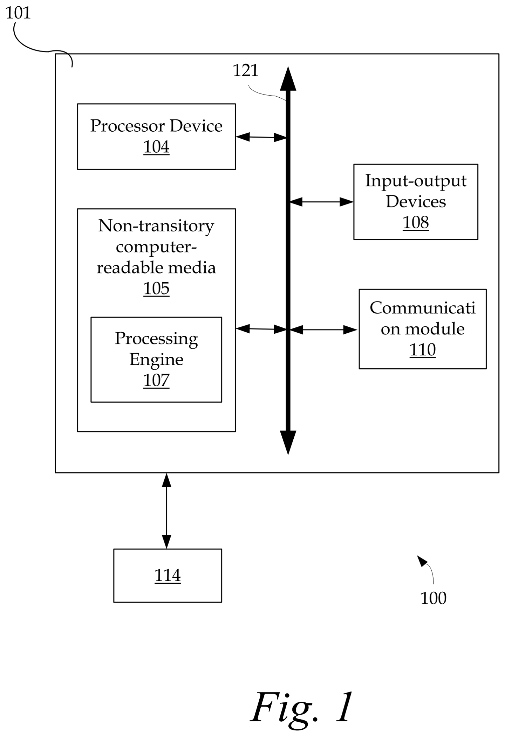

FIG. 1 is a block diagram illustrating an exemplary medical imaging system 100. In some implementations, system 100 includes a computer system 101 coupled to a medical imaging device 114. Computer system 101 includes a processor device 104 coupled to one or more non-transitory computer-readable media 105 (e.g., computer storage or memory device), input-output devices 108 (e.g., monitor, mouse, touchpad or keyboard) and communication module 110. Processor device 104 may include, for example, a central processing unit (CPU), a graphics processing unit (GPU), field-programmable gate array (FPGA), or a combination thereof. Computer system 101 may further include support circuits such as a cache, a power supply or battery, clock circuits, and a communications bus (not shown). Various other peripheral devices, such as client devices (e.g., workstations) and additional data storage devices and printing devices, may also be connected to the computer system 101.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 105. In particular, the present techniques may be implemented by a processing engine 107. Non-transitory computer-readable media 105 may include random access memory (RAM), read-only memory (ROM), magnetic floppy disk, flash memory, and other types of memories, or a combination thereof. The computer-readable program code is executed by processor device 104 to process data acquired by, for example, medical imaging device 114. The computer-readable program code is not intended to be limited to any particular programming language and implementation thereof. It will be appreciated that a variety of programming languages and coding thereof may be used to implement the teachings of the disclosure contained herein. The same or different computer-readable media 105 may be used for storing a database, including, but not limited to, image datasets, a knowledge base, individual subject data, medical records, diagnostic reports (or documents) for subjects, or a combination thereof.

Communication module 110 enables the computer system 101 to communicate with external systems and/or networks. In some implementations, communication module 110 includes a high-speed digital interface, such as Thunderbolt^{™}, universal serial bus (USB), multi-Gigabit Ethernet, optical fiber, waveguide technology, or a wireless interface. Other types of interfaces are also useful. In some implementations, communication module 110 includes wireless signal transceiver that communicates signals using a common communication protocol, such as Global System for Mobile Communications (GSM), WIFI, Bluetooth, Zigbee, LoRa, and TCP/IP.

Medical imaging device 114 is a radiological imaging device that acquires medical image data that reveals internal structures hidden by skin and bones of the subject. Such medical imaging device 114 may use technologies of X-ray radiography, computed tomography (CT), magnetic resonance (MR) imaging, ultrasound, endoscopy, elastography, tactile imaging, thermography, medical photography, nuclear medicine functional imaging techniques (e.g., positron emission tomography (PET), single-photon emission computed tomography (SPECT)), or a combination thereof.

It is to be further understood that, because some of the constituent system components and method steps depicted in the accompanying figures can be implemented in software, the actual connections between the systems components (or the process steps) may differ depending upon the manner in which the present framework is programmed. Given the teachings provided herein, one of ordinary skill in the related art will be able to contemplate these and similar implementations or configurations of the present framework.

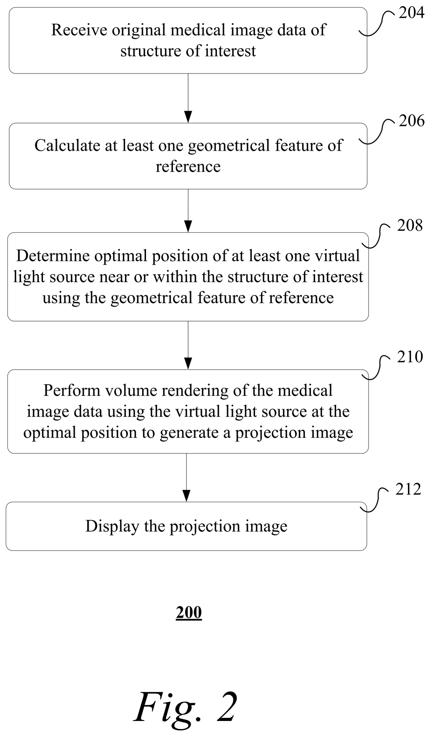

FIG. 2 shows an exemplary method 200 of medical image visualization. It should be understood that the steps of the method 200 may be performed in the order shown or a different order. Additional, different, or fewer steps may also be provided. Further, the method 200 may be implemented with system 100 of FIG. 1, a different system, or a combination thereof.

At 204, processing engine 107 receives original medical image data of at least one structure of interest. In some implementations, the original medical image data is a three-dimensional (3D) image data set (i.e., volumetric data) collected by, for example, an ultrasound scanner 114. Other numbers of dimensions (e.g., four-dimensional) and other imaging modalities may also be used. The structure of interest may be any anatomical structure (e.g., cardiac structure such as mitral valve, aortic valve, atria, ventricles) that is identified for further study.

A user interface may be provided to enable a user to select one or more structures of interest in the medical image data. In some implementations, an artificial intelligence (AI)-based analytical algorithm is independently applied to automatically detect and segment structures of interest. In other implementations, particularly in some scanning scenarios, such as 3D transesophageal echocardiography (TEE), the scanning protocols provide fairly consistent positions of structures of interest within the medical data.

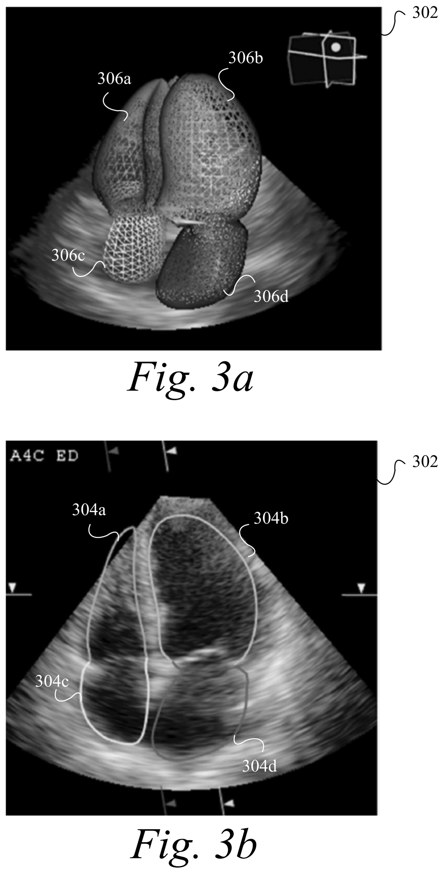

FIG. 3a shows an exemplary four-dimensional (4D) ultrasound cardiac image 302. Image 302 is multiplanar reconstructed (MPR) slice that represents an apical four-chamber view (A4C) of the heart. An artificial intelligence (AI)-based analytical algorithm is applied to the 4D ultrasound image 302 to generate meshes 306a-d corresponding to the right ventricle, the left ventricle, the left atrium and the right atrium of the heart. FIG. 3b shows the 4D ultrasound cardiac image 302 with intersections 304a-d corresponding to the generated meshes 306a-d.

Returning to FIG. 2, at 206, processing engine 107 calculates at least one geometrical feature of reference based on the medical image data. The geometrical feature of reference provides a point, line or plane of reference for placing the virtual light source. More than one geometrical feature may be determined. The geometrical feature of reference may be estimated approximately, since the lighting information is typically tolerant to minor inaccuracies in positions. A scaled-down (or down-sampled) representation of the original medical image data may be used for efficient computation of the geometrical feature of reference.

In some implementations, the geometrical feature of reference is a point, line or plane located at a pre-determined offset from the medical imaging device 114. For example, the point may be located at a pre-determined offset from the center of the face of an ultrasound transducer or probe. The pre-determined offset may be estimated from the distance between the center of the probe face to the structure of interest (e.g., mitral valve).

In other implementations, the geometrical feature is a centroid or center of mass of the structure of interest. For example, the centroid of a closed mesh representing the boundaries of the structure of interest (e.g., heart chamber) may be determined. The centroid may be determined by calculating, for instance, the average of positions of vertices of the closed mesh. The average of vertex positions may be weighted by triangular areas. The center of mass may be determined using, for example, physically-based estimation. Other types of geometrical features are also possible.

In yet other implementations, the geometrical feature of reference is an axis of a geometrical shape associated with the structure of interest. For example, the geometric shape (e.g., bounding box) encloses the structure of interest. The axis may be a centerline or one or more pre-defined anatomical axes. The geometric shape may be, for example, a cylinder, sphere, cube, polyhedron or tubular shape (e.g., cylinder, cone). In the case of tubular shapes (for e.g., blood vessels, valves), the geometrical feature may be the centerline axis. Skeletonization approaches from image morphology, such as thinning-based skeletonization, may be used to obtain centerlines of the geometrical shape. For complex geometrical shapes (e.g., spheroids, polyhedron), analytical techniques, such as principal component analysis, may be used to detect the geometrical feature of reference (e.g., longest axis).

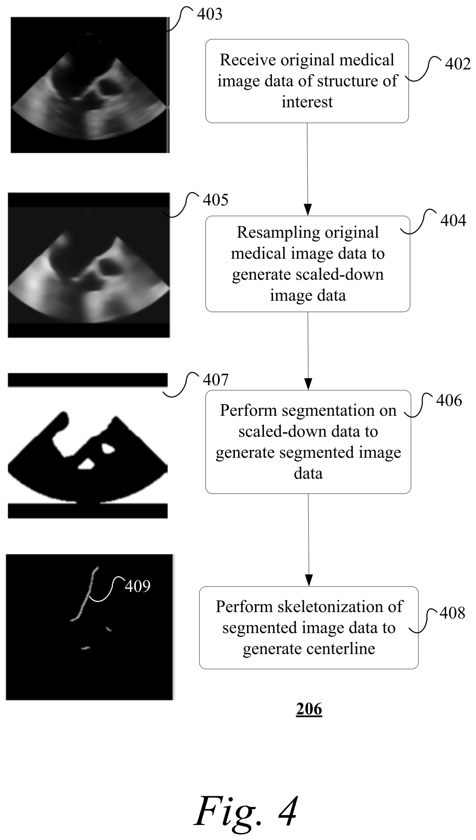

FIG. 4 shows an exemplary method 206 of automatically determining the centerline of the structure of interest. It should be understood that the steps of method 206 may be performed in the order shown or a different order. Additional, different, or fewer steps may also be provided. Further, the method 206 may be implemented with system 100 of FIG. 1, a different system, or a combination thereof.

At 402, processing engine 107 receives the original medical image data 303. An exemplary three-dimensional ultrasound cardiac image 303 is shown as an example. It should be appreciated that other types of images or anatomical structures of interest are also useful.

At 404, processing engine 107 resamples the original medical image data 303 to generate scaled-down image data 405. Resampling may be performed to reduce the resolution and size of the image data for faster processing. Resampling may be performed using average filtering. Smoothing techniques may be used to pre-filter the original medical image data 303 and remove high-frequency changes prior to resampling. Other types of downscaling algorithms that perform in real-time and introduce no high-frequency noise may also be used to generate a scaled-down representation of the original medical image data 303.

At 406, processing engine 107 performs segmentation on the scaled-down image data 405 to generate segmented image data 407. In some implementations, the segmented image data 407 is binary image data that is generated by a simple thresholding process that replaces each voxel in the scaled-down image data 405 with a white voxel if the image intensity is less than a fixed value called the threshold T, or a black voxel if the image intensity is greater than that threshold *T*. In the exemplary image data 407, this results in the heart structure becoming completely white, and the surrounding tissue becoming completely black. The threshold *T* may be determined by, for example, Otsu thresholding technique or histogram analysis. Other types of techniques may also be used.

At 406, processing engine 107 performs skeletonization of the segmented image data 407 to generate the centerline 409. Skeletonization reduces the binary image data 407 to centerlines, preserving the structure and topology for further analysis. Such skeletonization may be a thinning-based technique. For example, a GPU-based thinning technique may be used to efficiently compute centerlines in real-time. The thinning algorithm may iteratively remove the outer layer of voxels of the structure of interest until only a one-voxel-thin centerline is remaining. The centerlines also offer the possibility to be matched together from several consecutive data frames, thus providing coherence in the time domain.

Returning to FIG. 2, at 208, processing engine 107 determines the optimal position of at least one virtual light source using the at least one geometrical feature of reference. The virtual light source may be a point light source that emits light in all directions equally. Other types of light sources, such as a spot light source, a directional light source or area light source, may also be used. It should be appreciated that multiple point light sources or multiple types of light sources may be placed in the 3D medical image data space for rendering. For example, optimal positions of multiple different colored light sources may be determined.

The optimal position of the virtual light source may be at a pre-determined offset from the geometrical feature of reference, such that the virtual light source is positioned within or as close as possible to the structure of interest so as to minimize the amount of manual position adjustments to be made by the user. The user interface generated by processing engine 107 may enable the user to make final adjustments after the optimal position is automatically determined. The optimal position of the virtual light source may be automatically updated periodically, with the frequency of updates dependent upon the speed of detection of the structure of interest. The process that detects the structure of interest in the original medical image data may be performed independently from method 200.

In some implementations, the optimal position is determined at a fixed (or predetermined) offset from the centroid of the structure of interest. The offset may be provided by, for example, a user or clinical expert via the user interface. In other implementations, the optimal position is determined along the axis of a geometrical shape that encloses the structure of interest. For example, the optimal position of a virtual light source for a heart valve may be computed along the axis of a flat cylinder that encloses the heart valve. The optimal position may be located along the cylinder's axis at a pre-determined distance from the centroid of the cylinder. The pre-determined distance may be provided by, for example, a clinical expert or user via the user interface.

In yet other implementations, the optimal position is determined along the centerline of the structure of interest in order to enhance perception of the structure of interest. The optimal position may be located along the centerline at a pre-determined distance from the centroid of the structure of interest. The pre-determined distance may be provided by, for example, a clinical expert or user via the user interface.

Returning to FIG. 2, at 210, processing engine 107 performs volume rendering of the medical image data using the virtual light source at the optimal position to generate a projection image for display. The projection image represents a two-dimensional (2D) projection of the 3D medical image data that is used for display on a computer screen or other display device. The projection image is generated during volume rendering by mapping signal values in the 3D medical image data to particular illumination effects to assist in visualization.

Illumination effects may be determined by simulating the effect of the virtual light source at the optimal position by adding shadows, highlights and/or colors to provide illumination of the structure of interest in the projection image. In some implementations, illumination effects are stored in a light cache for speeding up rendering times. Volume rendering techniques may use the light cache to render the projection images for display. Examples of such volume rendering techniques, include, but are not limited to, ray-tracing, direct volume rendering, slicing, voxel cone tracing, or a combination thereof. For instance, GPU-accelerated ray-tracing may be used to compute exactly two bounces of global illumination supporting the light cache.

At 210, processing engine 107 displays the projection image. Processing engine 107 may cause a display device to display the projection image for visualization. The display device may be, for instance, a monitor, organic light-emitting diode (OLED) display, liquid crystal display (LCD), projector,
plasma display, cathode ray tube (CRT), printer, mobile device display, television, head-mounted display, and any other suitable device. The projection image may be reviewed and analyzed by a user (e.g., medical professional). The user may manipulate and/or choose the appropriate field-of-view and/or other visualization parameters.

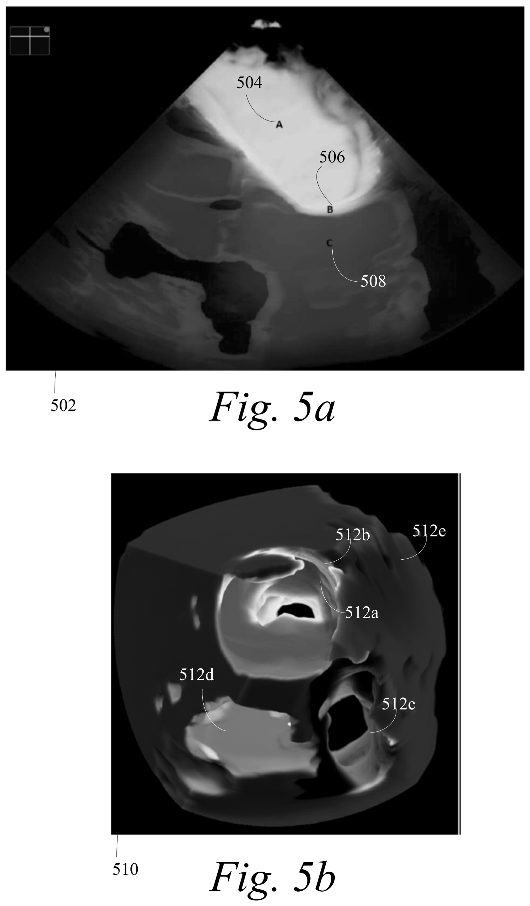

FIG. 5a shows an exemplary projection image 502 of a heart. The virtual light is positioned in the left atrium 504. The light from the virtual source does not enter the left ventricle 508 through the mitral valve 506. FIG. 5b shows another exemplary projection image 510 of a heart. Five virtual point lights are placed in chambers and arteries 512a-e of the heart. The five virtual point lights have different colors to facilitate visualization of the different areas of the heart.

The following is a list of non-limiting illustrative embodiments disclosed herein:
Illustrative embodiment 1. A method of visualization, comprising: receiving medical image data of at least one structure of interest; calculating at least one geometrical feature of reference based on the medical image data; determining an optimal position of at least one virtual light source near or within the structure of interest using the geometrical feature of reference; performing volume rendering of the medical image data using the at least one virtual light source at the optimal position to generate a projection image; and displaying the projection image.
Illustrative embodiment 2. The method of illustrative embodiment 1 comprising: providing a user interface to enable a user to select the at least one structure of interest.
Illustrative embodiment 3. The method of any one of illustrative embodiments 1-2 further comprises: resampling the medical image data to generate scaled-down image data, wherein the geometrical feature of interest is calculated based on the scaled-down image data.
Illustrative embodiment 4. The method of any one of illustrative embodiments 1-3 wherein calculating the at least one geometrical feature of interest comprises calculating a point, line or plane of reference located at a pre-determined offset from a medical imaging device.
Illustrative embodiment 5. The method of any one of illustrative embodiments 1-4 wherein calculating the at least one geometrical feature of interest comprises calculating a point, line or plane of reference of the at least one structure of interest.
Illustrative embodiment 6. The method of illustrative embodiment 5 wherein calculating the at least one geometrical feature of interest comprises determining a centroid or center of mass of the at least one structure of interest.
Illustrative embodiment 7. The method of illustrative embodiment 5 wherein calculating the at least one geometrical feature of interest comprises determining an axis of a geometrical shape that encloses the at least one structure of interest.
Illustrative embodiment 8. The method of illustrative embodiment 7 wherein determining the axis of the geometrical shape that encloses the at least one structure of interest comprises determining a centerline axis of a bounding box.
Illustrative embodiment 9. The method of illustrative embodiment 5 wherein calculating the at least one geometrical feature of interest comprises: resampling the medical image data to generated scaled-down image data; performing segmentation of the scaled-down image data to generate segmented image data; and performing skeletonization of the segmented image data to generate a centerline.
Illustrative embodiment 10. The method of illustrative embodiment 9 wherein performing segmentation of the scaled-down image data comprises performing a thresholding process to generate binary image data.
Illustrative embodiment 11. The method of illustrative embodiment 9 wherein performing skeletonization of the segmented image data comprises performing a thinning-based technique.
Illustrative embodiment 12. The method of illustrative embodiment 11 wherein performing the thinning-based technique comprises iteratively removing an outer layer of voxels of the at least one structure of interest until only a one-voxel-thin centerline is remaining.
Illustrative embodiment 13. The method of any one of illustrative embodiments 1-12 wherein determining the optimal position of the at least one virtual light source comprises determining the optimal position at a pre-determined offset from the geometrical feature of reference.
Illustrative embodiment 14. The method of illustrative embodiment 13 wherein determining the optimal position comprises locating the optimal position along a centerline of the at least one structure of interest and at a pre-determined offset from a centroid of the at least one structure of interest.
Illustrative embodiment 15. The method of any one of illustrative embodiments 1-14 further comprising: determining illumination effects of the at least one virtual light source at the optimal position; and storing the illumination effects in a light cache, wherein the volume rendering of the medical image data is performed using the light cache.
Illustrative embodiment 16. A system, comprising: a non-transitory memory device for storing computer readable program code; and a processor device in communication with the non-transitory memory device, the processor device being operative with the computer readable program code to perform steps including receiving medical image data of at least one structure of interest, calculating at least one geometrical feature of reference based on the medical image data, determining an optimal position of at least one virtual light source near or within the structure of interest using the geometrical feature of reference, performing volume rendering of the medical image data using the at least one virtual light source at the optimal position to generate a projection image, and displaying the projection image.
Illustrative embodiment 17. The system of illustrative embodiment 16 wherein the at least one virtual light source comprises a point light source, a spot light source, a directional light source, and areal light source, or a combination thereof.
Illustrative embodiment 18. The system of any one of illustrative embodiments 16-17 wherein the at least one virtual light source comprises multiple different colored light sources.
Illustrative embodiment 19. The system of any one of illustrative embodiments 16-18 wherein the processor device is operative with the computer readable program code to calculate the at least one geometrical feature of interest based on a scaled-down representation of the medical image data.
Illustrative embodiment 20. One or more non-transitory computer-readable media comprising computer-readable instructions, that when executed by a processor device, cause the processor device to perform steps comprising: receiving medical image data of at least one structure of interest; calculating at least one geometrical feature of reference based on the medical image data; determining an optimal position of at least one virtual light source near or within the structure of interest using the geometrical feature of reference; performing volume rendering of the medical image data using the at least one virtual light source at the optimal position to generate a projection image; and displaying the projection image.

While the present framework has been described in detail with reference to exemplary embodiments, those skilled in the art will appreciate that various modifications and substitutions can be made thereto without departing from the spirit and scope of the invention as set forth in the appended claims. For example, elements and/or features of different exemplary embodiments may be combined with each other and/or substituted for each other within the scope of this disclosure and appended claims.

## Claims

1. A method (200) of visualization, comprising:
receiving (204) medical image data of at least one structure of interest;
calculating (206) at least one geometrical feature of reference based on the medical image data;
determining (208) an optimal position of at least one virtual light source near or within the structure of interest using the geometrical feature of reference; and
performing volume rendering (210) of the medical image data using the at least one virtual light source at the optimal position to generate a projection image.

2. The method (200) of claim 1 comprising:
providing a user interface to enable a user to select the at least one structure of interest.

3. The method (200) of claim 1 or 2 further comprises:
resampling (404) the medical image data to generate scaled-down image data, wherein the geometrical feature of interest is calculated based on the scaled-down image data.

4. The method (200) of one of claims 1 to 3 wherein calculating (206) the at least one geometrical feature of interest comprises calculating a point, line or plane of reference located at a pre-determined offset from a medical imaging device.

5. The method (200) of one of claims 1 to 4 wherein calculating (206) the at least one geometrical feature of interest comprises calculating a point, line or plane of reference of the at least one structure of interest.

6. The method (200) of claim 5 wherein calculating (206) the at least one geometrical feature of interest comprises determining a centroid or center of mass of the at least one structure of interest.

7. The method (200) of claim 5 or 6 wherein calculating (206) the at least one geometrical feature of interest comprises determining an axis of a geometrical shape that encloses the at least one structure of interest.

8. The method (200) of claim 7 wherein determining the axis of the geometrical shape that encloses the at least one structure of interest comprises determining a centerline axis of a bounding box.

9. The method (200) of one of claims 5 to 8 wherein calculating (206) the at least one geometrical feature of interest comprises:
resampling (404) the medical image data (403) to generated scaled-down image data (405);
performing segmentation (406) of the scaled-down image data (405) to generate segmented image data (407); and
performing skeletonization (408) of the segmented image data to generate a centerline (409).

10. The method (200) of claim 9 wherein performing segmentation (406) of the scaled-down image data comprises performing a thresholding process to generate binary image data (407).

11. The method (200) of claim 9 or 10 wherein performing skeletonization (408) of the segmented image data (407) comprises performing a thinning-based technique.

12. The method (200) of claim 11 wherein performing the thinning-based technique comprises iteratively removing an outer layer of voxels of the at least one structure of interest until only a one-voxel-thin centerline is remaining.

13. The method (200) of one of claims 1 to 12 wherein determining (208) the optimal position of the at least one virtual light source comprises determining the optimal position at a pre-determined offset from the geometrical feature of reference.

14. The method (200) of claim 13 wherein determining the optimal position comprises locating the optimal position along a centerline of the at least one structure of interest and at a pre-determined offset from a centroid of the at least one structure of interest.

15. A system (100), comprising:
a non-transitory memory device (105) for storing computer readable program code; and
a processor device (104) in communication with the non-transitory memory device (105), the processor device (104) being operative with the computer readable program code to perform steps including
receiving (204) medical image data of at least one structure of interest,
calculating (206) at least one geometrical feature of reference based on the medical image data,
determining (208) an optimal position of at least one virtual light source near or within the structure of interest using the geometrical feature of reference, and
performing volume rendering (210) of the medical image data using the at least one virtual light source at the optimal position to generate a projection image.
